# DEMANDE DE BREVET EUROPEEN

(11) **EP 4 802 906 A1**
(43) Date de publication de la demande: **09.09.2026**
(21) Numéro de dépôt: 26161927.4
(22) Date de dépôt: 03.03.2026
(51) Int. Cl.: A23L 33/17, A23L 33/175, A61K 31/198

(54) **COMPOSITION NUTRITIONNELLE ENRICHIE EN CITRULLINE DESTINÉE À LUTTER CONTRE LA DÉNUTRITION**

(30) Priorité: 04.03.2025 FR 2502165
(71) Demandeur: Senes Solutions, 44240 Sucé-sur-Erdre (FR)
(72) Inventeur: BREYTON, Anne Esther, 44390 PETIT MARS (FR); LAPIERRE, Dominique, 44240 SUCE-SUR-ERDRE (FR); JOLIVET, Elise, 44240 SUCE-SUR-ERDRE (FR); GARNIER, Julien, 44390 PETIT MARS (FR)
(74) Mandataire: Aquinov

(57) **Abrégé**

La présente invention concerne une composition nutritionnelle comprenant au moins une source de protéines sous la forme de concentrat de protéines et/ou d'isolat de protéines, et au moins un acide aminé non protéinogène, la citrulline. L'invention vise également des utilisations de cette composition, notamment pour améliorer l'état nutritionnel et la fonction musculaire chez les personnes âgées, dénutries, ou malnutrie.

## Description

### Domaine technique

L'invention se rapporte au domaine des compléments alimentaires, des aliments fonctionnels et des produits nutritionnels spécialisés visant à soutenir la santé, notamment la santé musculaire et le métabolisme protéique. Plus particulièrement l'invention concerne une composition nutritionnelle comprenant au moins une source de protéines sous la forme de concentrat de protéines et/ou d'isolat de protéines, et au moins un acide aminé non protéinogène, la citrulline. L'invention vise également des utilisations de cette composition, notamment pour améliorer l'état nutritionnel et la fonction musculaire chez les personnes âgées, dénutries, ou malnutries.

### Etat de l'art

La dénutrition et la sarcopénie représentent des enjeux majeurs de santé publique, particulièrement chez les personnes âgées. La dénutrition, caractérisée par un déficit d'apport ou d'assimilation des nutriments essentiels, peut entraîner une diminution des capacités fonctionnelles, une augmentation des risques de chutes et une récupération plus lente après des hospitalisations ou des maladies aiguës. La sarcopénie, définie comme une perte progressive et généralisée de la masse et de la fonction musculaires, amplifie ces effets et peut conduire à une fragilité accrue, une diminution de la qualité de vie, ainsi qu'un risque accru de complications.

Face à ces défis, les approches nutritionnelles jouent un rôle central. L'intégration d'acides aminés spécifiques dans les stratégies nutritionnelles s'est avérée prometteuse pour stimuler la synthèse protéique musculaire et réduire les impacts de la dénutrition et de la sarcopénie. Dans ce contexte, la citrulline, notamment la L-citrulline, un acide aminé non protéinogène, a suscité un intérêt particulier.

La L-citrulline joue un rôle central dans le métabolisme de l'arginine et la synthèse de l'oxyde nitrique (NO), une molécule essentielle pour la vasodilatation et l'apport sanguin aux muscles. De plus, la L-citrulline offre des avantages uniques pour le métabolisme protéique, notamment chez les personnes âgées. Contrairement à l'arginine, qui est partiellement métabolisée par le foie, la L-citrulline échappe à la séquestration splanchnique et est convertie en arginine directement dans les reins, augmentant ainsi son efficacité pour stimuler la synthèse protéique et améliorer la récupération musculaire.

Des travaux antérieurs, notamment ceux décrits dans le brevet WO 2008/049984, ont mis en évidence l'intérêt thérapeutique de la L-citrulline dans le traitement des états de dénutrition. Celui-ci décrit spécifiquement un médicament à base de L-citrulline, conçu pour être administré de manière contrôlée afin de maximiser son effet anabolique et son rôle dans la stimulation de la synthèse protéique musculaire. Cependant, cette approche s'inscrit exclusivement dans le cadre d'un médicament, ce qui limite son utilisation à des indications médicales et un suivi médical précis. Or, il existe un besoin pour développer des solutions nutritionnelles destinées à un usage nutritionnel quotidien. Pour y parvenir, il existe un besoin pour de nouvelles solutions nutritionnelles, permettant d'être acceptées par les personnes en ayant besoin, pour un usage quotidien. La présente invention vise ainsi à améliorer l'appétence du produit, afin que les personnes en ayant besoin aient un apport quotidien optimal.

### Résumé de l'invention

Pour répondre à ce besoin, l'invention propose d'associer la citrulline avec au moins une source de protéines dans une composition nutritionnelle pour non seulement améliorer l'appétence et l'acceptabilité nutritionnelle des produits pour les seniors, mais aussi renforcer la réponse anabolique musculaire en combinant les effets synergiques des protéines et de la citrulline. Cette approche offre ainsi une solution nutritionnelle pratique, efficace et accessible, répondant à des besoins spécifiques tout en offrant une alternative aux approches purement médicamenteuses.

Ainsi, l'invention concerne une composition nutritionnelle comprenant :
▪ au moins une source de protéine, sous la forme de concentrat de protéines et/ou d'isolat de protéines, et
▪ au moins un acide aminé, à savoir la citrulline,
dans laquelle la fraction protéique de ladite composition présente un indice chimique inférieur à 125 et une teneur en azote supérieure à 9%. Préférentiellement, la fraction protéique de ladite composition présente un indice chimique inférieur à 100 et une teneur en azote supérieure à 9%.

La citrulline peut être choisie parmi plusieurs formes, notamment la L-citrulline ou le malate de citrulline, préférentiellement, la L-citrulline.

Avantageusement, la source de protéine est d'origine végétale, animale ou algale.

Lorsque la source est d'origine végétale, celle-ci est préférentiellement choisie parmi le groupe constitué des protéines de blé, de pois, de soja, de riz, de lupin, de lentilles, de chanvre, de quinoa, d'amande, de maïs, de tomate, de champignons et leur mélange.

Lorsque la source est d'origine animale, celle-ci est préférentiellement choisie parmi le groupe constitué de la caséine, les protéines sériques, le collagène, le blanc d'œuf et leur mélange.

Lorsque la source est d'origine algale, celle-ci est préférentiellement choisie parmi le groupe constitué de la spiruline, les peptides d'algues et leur mélange.

Selon un autre objet préféré, la composition comprend au moins un acide aminé branché. Celui-ci est alors très préférentiellement choisi parmi le groupe constitué de la leucine, l'iso-leucine, la valine et leur mélange.

Selon un autre objet préféré, la composition comprend au moins un ingrédient supplémentaire, celui-ci est très préférentiellement choisi parmi le groupe constitué de vitamines, minéraux, polyphénols, antioxydants et leur mélange.

De façon particulièrement avantageuse, la teneur en protéine totale de la composition est supérieure à 55%, très préférentiellement supérieure à 80%. La teneur en protéine peut notamment être déterminée selon la méthode d'analyse Kjeldahl (Official method of analysis of the A.O.C. 1975, 12th ed. W Horwitz, E.D., New-York, p. 15-60).

Selon un autre objet préféré, la quantité de citrulline représente entre 14 et 50% du poids total de la composition, plus préférentiellement entre 20% et 35%.

Enfin, selon un autre aspect, l'invention se rapporte également à l'utilisation de la composition selon l'un des quelconques modes de réalisation décrit pour améliorer l'appétence, les propriétés organoleptiques et la quantité d'aliments ingérée chez les personnes âgées et/ou dénutries.

Selon une variante, ladite composition peut également être utilisée pour améliorer l'efficacité anabolique et la biodisponibilité des nutriments, ainsi que pour améliorer la fonction vasculaire.

Préférentiellement, la quantité journalière de la citrulline administrée à un être humain peut être comprise entre 1 et 15g, plus préférentiellement entre 3 et 10g.

Cette composition nutritionnelle offre ainsi une approche innovante pour répondre aux besoins spécifiques des personnes âgées ou dénutries, en combinant les effets bénéfiques des protéines et de la citrulline. Cette composition permet ainsi d'améliorer son appétence et ses propriétés organoleptiques afin de faciliter l'acceptation du produit par les personnes âgées ou dénutries.

D'autre part, à l'inverse de l'enseignement de l'art antérieur, dont les compositions nutritionnelles visent un indice chimique élevé, notamment supérieur à 100 ou 125, la composition selon l'invention présente un indice chimique dégradé, inférieur à 125, préférentiellement inférieur à 100, tout en maintenant une bonne efficacité de ladite composition. En effet, l'enseignement conventionnel dans le domaine est de rechercher un indice chimique élevé pour garantir une qualité nutritionnelle optimale. Or, la composition selon la présente invention va à contre-courant de cet enseignement en démontrant qu'un indice chimique dégradé, combiné à la citrulline et une teneur en azote élevée, permet paradoxalement d'obtenir une meilleure efficacité globale grâce à une consommation accrue.

Ainsi, la combinaison spécifique des protéines et de la citrulline, associée à un indice chimique dégradé, à savoir inférieur à 125, préférentiellement inférieur à 100, et une teneur en azote supérieure à 9%, permet de maintenir une bonne efficacité du produit.

Cette composition apporte également une quantité d'azote suffisante, notamment apportée par la citrulline et les protéines, pour des personnes âgées ou dénutries permettant ainsi le maintien d'un état nutritionnel correct et d'une masse musculaire suffisante, alors que l'indice chimique des protéines apportées est inférieur à 125, préférentiellement inférieur à 100. En effet, un tel produit équivalent en termes d'apport d'azote avec un index chimique, notamment supérieur à 100 ou 125, et sans citrulline, sera moins appétent et donc ne sera pas ingéré dans sa totalité par les personnes âgées ou dénutries, limitant fortement l'apport quotidien d'azote et de protéines ingérées, conduisant à une dénutrition dans le temps et une dégradation de la masse musculaire.

D'autres caractéristiques et avantages ressortiront de la description détaillée de l'invention, des exemples qui vont suivre.

### Description détaillée de l'invention

### Définition

Par « composition nutritionnelle » au sens de l'invention, on entend un mélange de nutriments destiné à la consommation humaine en complément d'une alimentation journalière. Cette composition est formulée pour répondre aux besoins nutritionnels spécifiques des personnes âgées ou dénutries et peut se présenter sous diverses formes galéniques telles que des poudres, des boissons, des barres nutritionnelles ou des gels, et est conçue pour être utilisée comme complément alimentaire, aliment fonctionnel ou produit nutritionnel spécialisé. La composition vise à améliorer l'appétence et les propriétés organoleptiques du produit pour améliorer l'état nutritionnel d'une personne en ayant besoin.

Par « concentrat de protéines » au sens de l'invention, on entend une forme concentrée de protéines obtenue à partir d'une source alimentaire spécifique, telle que le lait, le soja, le pois ou d'autres sources végétales ou animales, par un processus d'extraction et de purification partielle. Ce produit contient généralement entre 60% et 80% de protéines en poids sec. Les pourcentages restants pouvant comprendre des lipides, des glucides, ou encore des minéraux. Les concentrats de protéines conservent une partie des composés bioactifs naturellement présents dans la source d'origine et augmentent la teneur en protéines tout en maintenant certaines caractéristiques organoleptiques et fonctionnelles du produit final.

Par « isolat de protéines » au sens de l'invention, on entend une forme hautement purifiée de protéines extraites d'une source alimentaire spécifique, telle que le lait, le soja, le pois ou d'autres sources végétales ou animales. Ce produit est obtenu par un processus d'extraction et de purification permettant l'obtention d'une teneur en protéines généralement supérieure à 90% en poids sec. Les isolats de protéines contiennent ainsi très peu de composés non protéiques, avec des quantités minimales de lipides, de glucides et de minéraux. Cette forme de protéine est caractérisée par sa pureté élevée, sa digestibilité accrue et son profil d'acides aminés concentré.

Par « fraction protéique » au sens de l'invention, on entend une fraction constituée exclusivement de protéines et de peptides, isolée ou concentrée à partir d'une ou plusieurs sources alimentaires. Plus spécifiquement, elle comprend l'ensemble des protéines et peptides présents dans la composition, qu'ils proviennent de concentrats, d'isolats, d'acides aminés, ou d'autres formes de protéines ajoutées.

Par « indice chimique » au sens de l'invention, on entend une mesure utilisée pour évaluer la qualité nutritionnelle d'une protéine ou d'une fraction protéique, basée sur sa composition en acides aminés essentiels. Celui-ci est calculé en comparant la concentration de chaque acide aminé essentiel dans la protéine étudiée à celle d'une protéine de référence, telle que la protéine Afssa 2007 présentant le profil suivant : Histidine 17 mg/g ; Isoleucine 59 mg/g ; Leucine 27 mg/g ; Valine 27 mg/g ; Lysine 45 mg/g ; Méthionine+Cystine 23 mg/g ; Phénylalaline+Tyrosine 41 mg/g ; Thréonine 25 mg/g ; Tryptophane 6 mg/g. Par mg/g on entend milligramme d'acide aminé par gramme de protéine (ref : Apport en protéines : consommation, qualité, besoins et recommandations », AFSSA, 2007, p210). L'indice chimique est alors exprimé en pourcentage et est déterminé par l'acide aminé essentiel limitant, c'est-à-dire celui ayant le ratio le plus bas par rapport à la protéine de référence. Un indice chimique de 100 indique que la protéine contient tous les acides aminés essentiels dans des proportions au moins égales à celles de la protéine de référence. Un indice inférieur à 100 signifie qu'au moins un acide aminé essentiel est présent en quantité moindre que dans la référence. Ainsi, plus l'indice chimique est élevé, plus la protéine est considérée comme complète et de haute qualité nutritionnelle. Un indice inférieur à 100 suggère que la protéine pourrait être limitante pour au moins un acide aminé essentiel.

Par « acides aminés branchés », également connus sous l'acronyme BCAA (Branched-Chain Amino Acids), au sens de l'invention, on entend un groupe spécifique d'acides aminés essentiels caractérisés par leur structure moléculaire unique. Ils sont nommés "branchés" en raison de leur chaîne latérale aliphatique ramifiée, qui leur confère des propriétés physico-chimiques et métaboliques distinctes. Ces acides aminés sont considérés comme essentiels car ils ne peuvent pas être synthétisés par l'organisme humain et doivent être apportés par l'alimentation. Les BCAA sont principalement métabolisés dans les muscles squelettiques plutôt que dans le foie.

Par « appétence » au sens de l'invention, on entend la qualité d'un aliment ou d'une composition nutritionnelle qui stimule l'appétit et incite à la consommation. Elle se réfère donc à l'attrait global d'un aliment, englobant ses propriétés organoleptiques telles que le goût, l'odeur, la texture et l'apparence visuelle. L'appétence va également influencer la satisfaction gustative à l'aliment, déclenchant des mécanismes physiologiques qui stimulent l'envie de manger et la satisfaction lors de la consommation. Enfin, elle peut être modulée par divers facteurs, tels que la présence d'arômes naturels ou artificiels, la texture, la température, et la présentation du produit.

Par « propriétés organoleptiques » au sens de l'invention, on entend les caractéristiques sensorielles d'un aliment ou d'une composition nutritionnelle qui peuvent être perçues et évaluées par les organes des sens humains, telles que le goût (saveurs perçues par les papilles gustatives), l'odeur (arômes détectés par l'odorat), la texture (sensations tactiles en bouche), l'apparence visuelle (couleur, forme, brillance) et le son (lors de la mastication ou de la manipulation).

### Composition nutritionnelle

La présente invention a donc pour objet une composition nutritionnelle comprenant au moins une source de protéine sous la forme de concentrat et/ou d'isolat de protéines, et un acide aminé non protéinogène, la citrulline. Celle-ci peut se présenter sous diverses formes, notamment la L-citrulline ou le malate de citrulline. Préférentiellement, la citrulline est la L-citrulline. Cette composition est particulièrement adaptée pour répondre aux besoins nutritionnels spécifiques des personnes âgées ou dénutries.

De façon surprenante, la composition nutritionnelle selon la présente invention permet une consommation supérieure en acide aminé du fait de caractéristiques organoleptiques fortement améliorées. A titre d'exemple, l'association des protéines de poisson à un vecteur alimentaire de type compote est rédhibitoire pour un consommateur. La mise en œuvre de la composition selon l'invention permet ainsi une plus grande variété d'applications alimentaires et culinaires que ne le permettent les protéines seules, y compris la citrulline seule.

La citrulline est un acide aminé non protéinogène, ce qui signifie qu'elle n'est pas utilisée par l'organisme pour la synthèse des protéines. C'est un composé intermédiaire du cycle de l'urée et la forme L-citrulline est un précurseur de la L-arginine. La L-citrulline se caractérise par sa structure chimique unique, comprenant une chaîne latérale contenant un groupe uréido. Elle peut être synthétisée de manière endogène dans l'organisme, principalement dans l'intestin grêle, ou obtenue par l'alimentation, notamment à partir de certains fruits comme la pastèque. Contrairement à d'autres acides aminés, la L-citrulline n'est pas métabolisée de manière significative par le foie lors de son premier passage, ce qui lui permet d'atteindre la circulation systémique en concentrations plus élevées. Dans l'organisme, la L-citrulline est convertie en L-arginine principalement dans les reins, ce qui contribue à la production d'oxyde nitrique (NO), un vasodilatateur important. Enfin, la présence de L-citrulline permet d'optimiser la biodisponibilité des acides aminés essentiels dans la composition nutritionnelle, renforçant également la synthèse protéique musculaire.

La fraction protéique de la composition présente un indice chimique inférieur à 125, préférentiellement inférieur à 100, et une teneur en azote supérieure à 9%. Ceci permet ainsi d'obtenir une source de protéines de haute qualité nutritionnelle tout en maintenant une concentration élevée en acides aminés essentiels. Avantageusement, un tel indice chimique, notamment inférieur à 100 est tout aussi efficace en termes d'efficacité nutritionnelle au regard de la prise de masse musculaire qu'une composition nutritionnelle comprenant exclusivement des protéines avec un Indice Chimique (IC) supérieur ou égal à 100. Ceci s'explique par la quantité consommée de la composition comprenant les protéines et la citrulline qui devient supérieure à la quantité habituellement consommée et parce que l'efficacité anabolique apportée avec la même quantité d'azote est supérieure. En effet, la citrulline échappe à la séquestration splanchnique et est donc entièrement disponible pour agir sur les voies de synthèse protéique.

La source de protéine peut être d'origine végétale, animale ou algale. Cette diversité de sources permet d'adapter la composition nutritionnelle aux préférences et aux besoins spécifiques des consommateurs, notamment des personnes âgées dénutries, tout en offrant une flexibilité dans la formulation. Les protéines végétales, avantageusement choisies parmi celles issues du blé, du pois, du soja, du riz, du lupin, des lentilles, du chanvre, du quinoa, de l'amande, du maïs, de la tomate, des champignons et leur mélange, peuvent être particulièrement intéressantes pour leur profil nutritionnel et leur durabilité. Les protéines animales avantageusement choisies parmi la caséine, les protéines sériques, le collagène ou le blanc d'œuf et leur mélange, offrent une excellente digestibilité et un profil d'acides aminés complet. Les protéines algales avantageusement choisies parmi la spiruline ou les peptides d'algues, peuvent apporter des nutriments uniques et contribuer à la diversification des sources protéiques.

La composition selon la présente invention comprend préférentiellement des acides aminés branchés afin de stimuler la synthèse protéique musculaire. Très préférentiellement, l'acide aminé branché est choisi parmi le groupe constitué de la leucine, l'iso-leucine, la valine et leur mélange.

Selon un autre objet préféré, la composition selon l'invention comprend des vitamines, et/ou des minéraux, et/ou des polyphénols, et/ou des antioxydants. L'ajout de ces composants permet d'enrichir le profil nutritionnel de la composition et de cibler des besoins spécifiques. Ainsi, les vitamines et minéraux soutiennent diverses fonctions physiologiques. Les polyphénols et antioxydants peuvent contribuer à réduire le stress oxydatif et l'inflammation impliqués dans le vieillissement et la dénutrition.

Préférentiellement, la teneur en protéine totale de la composition est supérieure à 55%, préférentiellement supérieure à 80%. Cette concentration élevée en protéines permet de fournir un apport protéique significatif dans un volume réduit, ce qui est particulièrement avantageux pour les personnes ayant des difficultés à consommer de grandes quantités d'aliments, telles que les personnes âgées.

La quantité de citrulline, notamment la L-citrulline représente préférentiellement entre 14 et 50% du poids total de la composition, plus préférentiellement entre 20% et 35%. Cette proportion de citrulline est conçue pour fournir une dose efficace tout en maintenant un équilibre avec les autres composants de la formulation. La citrulline joue un rôle important dans le métabolisme de l'arginine et la synthèse de l'oxyde nitrique, contribuant ainsi à améliorer la fonction vasculaire et la synthèse protéique musculaire.

Selon un autre objet, la composition nutritionnelle peut être formulée sous diverses formes galéniques, telles que des poudres, des boissons, des barres nutritionnelles ou des gels. Cette variété de formes permet de s'adapter aux préférences et aux capacités de consommation de chacun des consommateurs. En outre, elle permet de proposer des aliments, des suppléments nutritionnels, ou encore des denrées alimentaires destinées à des fins médicales spéciales (DADFMS).

La composition nutritionnelle selon l'invention peut être préparé selon les connaissances de l'homme du métier, notamment selon un procédé comprenant les étapes suivantes :
a. sélectionner une ou plusieurs sources de protéines sous forme de concentrat et/ou d'isolat de protéines.
b. éventuellement, préparer la L-citrulline sous forme de poudre,
c. éventuellement, sélectionner les ingrédients supplémentaires (acides aminés branchés, vitamines, minéraux, polyphénols, antioxydants),
d. préparer les ingrédients sélectionnés dans les quantités appropriées pour atteindre la teneur en protéine totale désirée et la proportion en L-citrulline souhaitée,
e. mélanger à sec les protéines et la L-citrulline ou le malate de citrulline dans un mélangeur approprié,
f. éventuellement, incorporer les acides aminés branchés, vitamines, minéraux, polyphénols et/ou antioxydants si nécessaire,
g. mélanger à nouveau pour assurer une répartition uniforme de tous les composants,
h. vérifier les teneurs en protéines, L-citrulline et autres nutriments, ainsi que l'indice chimique de la fraction protéique et la teneur en azote.

Ce procédé peut être adapté par l'homme du métier en fonction des spécificités de chaque formulation et des équipements disponibles. Il permet d'obtenir une composition nutritionnelle homogène, avec les proportions désirées de protéines et de citrulline, tout en répondant aux besoins de l'invention et assurant la qualité et la stabilité du produit final.

### Utilisation

Selon un autre aspect, la présente composition selon l'invention est utilisée pour améliorer l'appétence, les propriétés organoleptiques et la quantité d'aliments ingérés chez les personnes âgées et/ou dénutries. Cet aspect est particulièrement important car il contribue fortement à augmenter l'apport nutritionnel global chez ces populations à risque de malnutrition.

Les propriétés organoleptiques jouent un rôle crucial dans l'acceptabilité, l'appétence et la consommation régulière du produit. Les différentes propriétés organoleptiques interagissent entre elles, créant alors une expérience sensorielle globale. Ces propriétés peuvent être modifiées ou optimisées pour répondre aux préférences spécifiques de certaines populations, comme les personnes âgées ou dénutries. En outre, les propriétés organoleptiques influencent les réponses physiologiques comme la sécrétion salivaire et gastrique, améliorant ainsi la digestion et l'absorption des nutriments.

Ainsi, l'invention vise une utilisation de la composition selon l'une des quelconques modes de réalisation précédemment décrit pour :
▪ améliorer l'appétence et les propriétés organoleptiques de produits alimentaires destinés aux personnes âgées et ou dénutries, et/ou
▪ améliorer/augmenter la quantité d'aliment ingérée lors des repas des personnes âgées et ou dénutries, et/ou
▪ améliorer l'efficacité anabolique de produits alimentaires destinés aux personnes âgées et ou dénutries, et/ou
▪ améliorer la biodisponibilité des nutriments destinés aux personnes âgées et ou dénutries, et/ou
▪ améliorer la fonction vasculaire.

L'amélioration de la fonction vasculaire, en particulier la production accrue d'oxyde nitrique améliore l'apport sanguin et l'oxygénation des tissus musculaires, réduisant ainsi la fatigue et favorisant la récupération.

Selon un autre objet, la composition est également destinée à être utilisée pour améliorer l'efficacité anabolique et la biodisponibilité des nutriments. La combinaison de protéines de haute qualité et de la citrulline favorise une meilleure utilisation des acides aminés pour la synthèse protéique, contribuant ainsi au maintien de la masse musculaire.

La quantité journalière de citrulline, notamment la L-citrulline, administrée à un être humain est avantageusement comprise entre 1 et 15g. Chez l'adulte, la consommation de 2, 5, 10 ou 15 g de citrulline est très bien tolérée et induit une augmentation du taux d'arginine dans le sang et du bilan azoté. Plus préférentiellement, la quantité journalière de citrulline administrée à un être humain est comprise entre 3 et 10g. Cette plage de dosage permet d'être ajustée en fonction des besoins spécifiques.

En combinant les effets bénéfiques des protéines et de la citrulline, cette composition nutritionnelle offre une approche innovante pour répondre aux besoins spécifiques des personnes âgées ou dénutries. Elle contribue à améliorer leur état nutritionnel, leur fonction musculaire et leur qualité de vie globale.

Elle offre ainsi une alternative nutritionnelle adaptée aux besoins des personnes âgées : avec des produits appétents, digestes et adaptés aux régimes spécifiques (omnivores ou végétariens). A quantité d'acides aminés identiques l'apport d'une composition comprenant la citrulline et des protéines animales ou végétales augmentera de façon plus importante la masse musculaire qu'une quantité d'acide aminés apportés uniquement par des protéines animales ou végétales.

L'invention est à présent illustrée par des exemples non limitatifs de compositions selon l'invention et par des résultats.

### Exemples

### Exemple 1

Un premier exemple de composition nutritionnelle selon l'invention est détaillé dans le tableau 1, ci-après.

**[Tableau 1]**

| **Ingrédients** | **(g/100g)** |
|---|---|
| Isolat de protéine de pois | 55,9 |
| Hydrolysat de protéine de blé | 30,1 |
| L-Citrulline | 14 |
| Total (g/100g) | 100 |

| **Informations nutritionnelles :** | |
|---|---|
| Teneur en Azote | 14,3 |
| Teneur en protéines selon la méthode Nx6,25 | 89,3 |
| Teneur en protéines (g/100g) non citrulline | 68,3 |
| Teneur en L-Citrulline (g/100g) | 14 |
| Indice chimique | 93 |
| Portion quotidienne (g/jour) | 25 |

| Apport quotidien de la portion : | |
|---|---|
| En protéine non citrulline (g/j) | 17 |
| En L-citrulline (g/j) | 3,5 |

Cette composition est préparée selon les connaissances de l'homme du métier, tel que le procédé décrit précédemment dans la présente description.

### Exemple 2

Un autre exemple de composition nutritionnelle selon l'invention est détaillé dans le tableau 2, ci-après.

**[Tableau 2]**

| **Ingrédients** | **(g/100g)** |
|---|---|
| Isolat de protéine de pois | 46,8 |
| Hydrolysat de protéine de blé | 25,2 |
| L-Citrulline | 28 |
| Total (g/100g) | 100 |

| **Informations nutritionnelles :** | |
|---|---|
| Teneur en Azote | 15,9 |
| Teneur en protéines selon la méthode Nx6,25 | 99,4 |
| Teneur en protéines (g/100g) non citrulline | 57,6 |
| Teneur en L-Citrulline (g/100g) | 28 |
| Indice chimique | 75 |
| Portion quotidienne (g/jour) | 25 |

| Apport quotidien de la portion : | |
|---|---|
| En protéine non citrulline (g/j) | 14 |
| En L-citrulline (g/j) | 7 |

Cette composition est préparée selon les connaissances de l'homme du métier, tel que le procédé décrit précédemment.

### Exemple 3

Un autre exemple de composition nutritionnelle selon l'invention est détaillé dans le tableau 3, ci-après.

**[Tableau 3]**

| **Ingrédients** | **(g/100g)** |
|---|---|
| Isolat de protéine de pois | 39 |
| Hydrolysat de protéine de blé | 21 |
| L-Citrulline | 40 |
| Total (g/100g) | 100 |

| **Informations nutritionnelles :** | |
|---|---|
| Teneur en Azote | 17,2 |
| Teneur en protéines selon la méthode Nx6,25 | 107,6 |
| Teneur en protéines (g/100g) non citrulline | 48 |
| Teneur en L-Citrulline (g/100g) | 40 |
| Indice chimique | 61 |
| Portion quotidienne (g/jour) | 25 |

| Apport quotidien de la portion : | |
|---|---|
| En protéine non citrulline (g/j) | 12 |
| En L-citrulline (g/j) | 10 |

Cette composition est préparée selon les connaissances de l'homme du métier, tel que le procédé décrit précédemment.

### Exemple 4

Un autre exemple de composition nutritionnelle selon l'invention est détaillé dans le tableau 4, ci-après.

**[Tableau 4]**

| **Ingrédients** | **(g/100g)** |
|---|---|
| Collagène hydrolysé | 72 |
| L-Citrulline | 28 |
| Total (g/100g) | 100 |

| **Informations nutritionnelles :** | |
|---|---|
| Teneur en azote (g/100g) | 18,5 |
| Teneur en protéines selon la méthode Nx6,25 | 115,8 |
| Teneur en protéines (g/100g) non citrulline | 65,52 |
| Teneur en L-Citrulline (g/100g) | 28 |
| Indice chimique | 0 |
| Portion quotidienne (g/jour) | 25 |

| Apport quotidien de la portion : | |
|---|---|
| En protéine non citrulline (g/j) | 16 |
| En L-citrulline (g/j) | 7 |

Cette composition est préparée selon les connaissances de l'homme du métier, tel que le procédé décrit précédemment.

### Exemple 5

Un autre exemple de composition nutritionnelle selon l'invention est détaillé dans le tableau 5, ci-après.

**[Tableau 5]**

| **Ingrédients** | **(g/100g)** |
|---|---|
| Isolat de Caséine | 86 |
| L-Citrulline | 14 |
| Total (g/100g) | 100 |

| **Informations nutritionnelles :** | |
|---|---|
| Teneur en azote (g/100g) | 15,8 |
| Teneur en protéines selon la méthode Nx6,25 | 98,5 |
| Teneur en protéines (g/100g) non citrulline | 79,12 |
| Teneur en L-Citrulline (g/100g) | 14 |
| Indice chimique | 122 |
| Portion quotidienne (g/jour) | 25 |

| Apport quotidien de la portion : | |
|---|---|
| En protéine non citrulline (g/j) | 20 |
| En L-citrulline (g/j) | 3,5 |

Cette composition est préparée selon les connaissances de l'homme du métier, tel que le procédé décrit précédemment.

### Exemple 6

Un autre exemple de composition nutritionnelle selon l'invention est détaillé dans le tableau 6, ci-après.

**[Tableau 6]**

| **Ingrédients** | **(g/100g)** |
|---|---|
| Caséine | 72 |
| L-Citrulline | 28 |
| Total (g/100g) | 100 |

| **Informations nutritionnelles :** | |
|---|---|
| Teneur en azote (g/100g) | 17,1 |
| Teneur en protéines selon la méthode Nx6,25 | 106,9 |
| Teneur en protéines (g/100g) non citrulline | 66,24 |
| Teneur en L-Citrulline (g/100g) | 28 |
| Indice chimique | 101 |
| Portion quotidienne (g/jour) | 25 |

| Apport quotidien de la portion : | |
|---|---|
| En protéine non citrulline (g/j) | 17 |
| En L-citrulline (g/j) | 7 |

Cette composition est préparée selon les connaissances de l'homme du métier, tel que le procédé décrit précédemment.

### Exemple 7

Un autre exemple de composition nutritionnelle selon l'invention est détaillé dans le tableau 7, ci-après.

**[Tableau 7]**

| **Ingrédients** | **(g/100g)** |
|---|---|
| Isolat de Caséine | 70 |
| L-Citrulline | 30 |
| Total (g/100g) | 100 |

| **Informations nutritionnelles :** | |
|---|---|
| Teneur en azote (g/100g) | 17,3 |
| Teneur en protéines selon la méthode Nx6,25 | 108,1 |
| Teneur en protéines (g/100g) non citrulline | 64,40 |
| Teneur en L-Citrulline (g/100g) | 30 |
| Indice chimique | 98 |
| Portion quotidienne (g/jour) | 25 |

| Apport quotidien de la portion : | |
|---|---|
| En protéine non citrulline (g/j) | 16 |
| En L-citrulline (g/j) | 7,5 |

Cette composition est préparée selon les connaissances de l'homme du métier, tel que le procédé décrit précédemment.

### Exemple 8

Un autre exemple de composition nutritionnelle selon l'invention est détaillé dans le tableau 8, ci-après.

**[Tableau 8]**

| **Ingrédients** | **(g/100g)** |
|---|---|
| Isolat de Caséine | 60 |
| L-Citrulline | 40 |
| Total (g/100g) | 100 |

| **Informations nutritionnelles :** | |
|---|---|
| Teneur en azote (g/100g) | 18,2 |
| Teneur en protéines selon la méthode Nx6,25 | 114,1 |
| Teneur en protéines (g/100g) non citrulline | 55,20 |
| Teneur en L-Citrulline (g/100g) | 40 |
| Indice chimique | 83 |
| Portion quotidienne (g/jour) | 25 |

| Apport quotidien de la portion : | |
|---|---|
| En protéine non citrulline (g/j) | 14 |
| En L-citrulline (g/j) | 10 |

Cette composition est préparée selon les connaissances de l'homme du métier, tel que le procédé décrit précédemment.

### Exemple 9

Un autre exemple de composition nutritionnelle selon l'invention est détaillé dans le tableau 9, ci-après.

**[Tableau 9]**

| **Ingrédients** | **(g/100g)** |
|---|---|
| Isolat de protéine de pois | 20,8 |
| Hydrolysat de protéine de blé | 11,2 |
| L-Citrulline | 28 |
| Total (g/100g) | 60 |

| **Informations nutritionnelles :** | |
|---|---|
| Teneur en azote (g/100g) | 10,8 |
| Teneur en protéines selon la méthode Nx6,25 | 67,4 |
| Teneur en protéines (g/100g) non citrulline | 25,42 |
| Teneur en L-Citrulline (g/100g) | 28 |
| Indice chimique | 53 |
| Portion quotidienne (g/jour) | 25 |

| Apport quotidien de la portion : | |
|---|---|
| En protéine non citrulline (g/j) | 6 |
| En L-citrulline (g/j) | 7 |

Cette composition est préparée selon les connaissances de l'homme du métier, tel que le procédé décrit précédemment.

### Essai d'efficacité d'une composition nutritionnelle selon l'invention

Une étude consommateur a été mise en place visant à étudier l'effet d'une composition selon l'invention, à savoir une supplémentation en protéines végétales associées à de la L-citrulline chez des personnes âgées dénutries en institution.

Le premier objectif de l'étude est d'évaluer l'efficacité de la supplémentation en protéines végétales associées à de la L-citrulline sur la masse musculaire chez des personnes âgées dénutries en institution. Dans un second temps, l'étude vise à évaluer l'effet de cette supplémentation sur la vitalité cellulaire du patient, son hygiène de vie, ses apports protéiques (quantitatif et qualitatif), son taux de masse grasse, sa santé osseuse et son hydratation.

Une étude a ainsi été mise en place. Suite à une randomisation, un groupe est attribué au patient et ce groupe détermine l'ordre dans lequel le patient va consommer les produits. Il débute son étude soit avec la consommation de 2 portions de 12,5g de protéine végétale à base de blé et de pois, soit avec la consommation de 2 portions de 12,5g (protéine végétale + L-Citrulline telle que décrit dans l'exemple n°2) et change de produit à la suite de la période de Wash-Out.

Dans le cadre de cette étude, 20 personnes âgées de plus de 70 ans et souffrant de dénutrition modérée ont été inclues. Les deux produits de l'étude ont été consommés durant 1 mois chacun. L'étude comportait 4 visites au cours desquelles, des évaluations de la quantité d'ingesta (par ingesta on entend les substances alimentaires solides ou liquides ingérées, c'est-à-dire absorbées par l'organisme par ingestion par voie orale), des mesures anthropométriques, des mesures de bio-impédancemétrie et des questionnaires de qualité de vie ont été réalisées afin de suivre l'évolution des différents objectifs cités plus haut.

Le relevé de la quantité d'ingesta permet d'évaluer la prise alimentaire, et l'acceptation des produits par le mangeur, et leur appréciation des aliments.

La bio-impédancemétrie permet de mesurer la composition corporelle d'une personne et donc d'avoir accès à son statut nutritionnel, son statut métabolique et son statut hydrique.

Les résultats sont présentés dans les tableaux 10 et 11, ci-après.

**[Tableau 10]**

| **Ingrédients** | **Produit 1 (recette de l'exemple n°2) (g/100g)** | **Produit 2 g/100g** |
|---|---|---|
| Isolat de protéine de pois | 46,8 | 65 |
| Hydrolysat de protéine de blé | 25,2 | 35 |
| L-Citrulline | 28 | 0 |
| Total (g/100g) | 100 | 100 |

| **Informations nutritionnelles :** | **Produit 1** | **Produit 2** |
|---|---|---|
| Teneur en Azote (g/100g) | 15,9 | 12,7 |
| Teneur en protéines selon la méthode Nx6,25 | 99,2 | 80 |
| Teneur en protéines (g/100g) non citrulline | 57,6 | 80 |
| Teneur en L-Citrulline (g/100g) | 28 | 0 |
| Indice chimique | 75 | 112 |
| Portion quotidienne (g/jour) | 25 | 25 |

| Apport quotidien de la portion : | | |
|---|---|---|
| En protéine non citrulline (g/j) | 14,4 | 20 |
| En L-citrulline (g/j) | 7 | 0 |

**[Tableau 11]**

| | **Produit 1** | **Produit 2** |
|---|---|---|
| Observation de la quantité d'aliment ingérée sur une semaine (% de l'assiette servie réellement ingéré) | 83,75% | 66,25% |
| Consommation Moyenne quotidienne de protéines non citrulline (g/j) | 12,1 | 13,3 |
| Consommation réelle moyenne quotidienne d'azote (g/j) | 13,3 | 8,4 |

Les résultats montrent une meilleure consommation des repas de manière générale. L'augmentation de la consommation quotidienne d'azote entraine également une amélioration du statut nutritionnel des patients et une amélioration de la masse musculaire. Il en résulte que la consommation du produit composé de (protéine végétale + L-Citrulline) permet le maintien d'un état nutritionnel correct et d'une masse musculaire suffisante pour des personnes âgées ou dénutries répondant ainsi aux besoins de la présente invention.

## Revendications

1. Composition nutritionnelle comprenant :
• au moins une source de protéine sous la forme de concentrat de protéines et/ou d'isolat de protéines, et
• au moins un acide aminé, à savoir la citrulline,
**caractérisée en ce que** la fraction protéique de ladite composition présente un indice chimique inférieur à 125 et une teneur en azote supérieure à 9%.

2. Composition selon la revendication précédente, dans laquelle l'indice chimique est inférieur à 100.

3. Composition selon l'une des revendications 1 ou 2, dans laquelle la source de protéine est d'origine végétale, animale ou algale.

4. Composition selon la revendication précédente, dans laquelle la source de protéine végétale est choisie parmi le groupe constitué des protéines de blé, pois, soja, riz, lupin, lentilles, chanvre, quinoa, amande, maïs, tomate, champignons et leur mélange.

5. Composition selon la revendication 3, dans laquelle la source de protéine animale est choisie parmi le groupe constitué de la caséine, les protéines sériques, le collagène, le blanc d'œuf et leur mélange.

6. Composition selon la revendication 3, dans laquelle la source de protéine algale est choisie parmi le groupe constitué de la spiruline, les peptides d'algues et leur mélange.

7. Composition selon l'une des revendications précédentes, comprenant en outre au moins un acide aminé branché, préférentiellement l'acide aminé branché est choisi parmi le groupe constitué de la leucine, l'iso-leucine, la valine et leur mélange.

8. Composition selon l'une des revendications précédentes, ladite composition comprend également au moins un ingrédient supplémentaire choisi parmi le groupe constitué de vitamines, minéraux, polyphénols, antioxydants et leur mélange.

9. Composition selon l'une des revendications précédentes, dans laquelle la teneur en protéine totale est supérieure à 55%, préférentiellement supérieur à 80%.

10. Composition selon l'une des revendications précédentes, dans laquelle la quantité de citrulline est comprise entre 14 et 50% en poids du poids total de la composition, préférentiellement entre 20% et 35%.

11. Utilisation d'une composition selon l'une des revendications précédentes, dans laquelle la quantité de citrulline administrée de façon journalière à un être humain est comprise entre 1 et 15g, préférentiellement entre 3 et 10g.

12. Utilisation d'une composition selon l'une des revendications 1 à 10 pour améliorer l'appétence et les propriétés organoleptiques de produits alimentaires destinés aux personnes âgées et/ou dénutries.

13. Utilisation d'une composition selon l'une des revendications 1 à 10 pour augmenter la quantité d'aliment ingérée lors des repas des personnes âgées et/ou dénutries

14. Utilisation d'une composition selon l'une des revendications 1 à 10, pour améliorer l'efficacité anabolique de produits alimentaires destinés aux personnes âgées et/ou dénutries.

15. Utilisation d'une composition selon l'une des revendications 1 à 10, pour améliorer la biodisponibilité des nutriments destinés aux personnes âgées et/ou dénutries ; et/ou pour améliorer la fonction vasculaire.
